Europäisches Patentamt

European Patent Office

Office européen des brevets

(19)

(11) **EP 0 979 222 B1**

(12) **EUROPÄISCHE PATENTSCHRIFT**

(45) Veröffentlichungstag und Bekanntmachung des Hinweises auf die Patenterteilung:
**13.02.2002 Patentblatt 2002/07**

(21) Anmeldenummer: **98922688.1**

(22) Anmeldetag: **11.04.1998**

(51) Int Cl.$^7$: **C07C 51/215**, C07C 51/25, C07C 53/08, B01J 23/68, B01J 23/652

(86) Internationale Anmeldenummer:
**PCT/EP98/02124**

(87) Internationale Veröffentlichungsnummer:
**WO 98/47850 (29.10.1998 Gazette 1998/43)**

(54) **KATALYSATOR UND VERFAHREN ZUR KATALYTISCHEN OXIDATION VON ETHAN ZU ESSIGSÄURE**

PROCESS AND CATALYST FOR PREPARING ACETIC ACID BY CATALYTIC OXIDATION OF ETHANE

CATALYSEUR ET PROCEDE DE PREPARATION D'ACIDE ACETIQUE PAR OXYDATION CATALYTIQUE D'ETHANE

(84) Benannte Vertragsstaaten:
**BE DE ES FR GB IE IT NL**

(30) Priorität: **23.04.1997 DE 19717076**

(43) Veröffentlichungstag der Anmeldung:
**16.02.2000 Patentblatt 2000/07**

(73) Patentinhaber: **Celanese GmbH**
**60439 Frankfurt (DE)**

(72) Erfinder:
• **BORCHERT, Holger**
**D-67278 Bockenheim (DE)**
• **DINGERDISSEN, Uwe**
**D-64342 Seeheim-Jugenheim (DE)**
• **ROESKY, Rainer**
**D-65929 Frankfurt (DE)**

(56) Entgegenhaltungen:
**EP-A- 0 407 091**      **EP-A- 0 480 594**
**EP-A- 0 620 205**      **EP-A- 0 801 979**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein Verfahren zur selektiven Herstellung von Essigsäure durch katalytische Gasphasenoxidation von Ethan in Gegenwart eines Wolfram enthaltenden Katalysators, sowie den Katalysator.

Die oxidative Dehydrierung von Ethan zu Ethylen in der Gasphase, bei Temperaturen > 500°C ist beispielsweise aus US-A-4 250 346, US-A-4 524 236 und US-A-4 568 790 bekannt. So wird in US-A-4 250 346 die Verwendung einer Katalysatorzusammensetzung offenbart, die die Elemente Molybdän, X und Y im Verhältnis a:b:c enthält, zur Umwandlung von Ethan in Ethylen, worin

X gleich Cr, Mn, Nb, Ta, Ti, V, und/oder W ist, und
Y gleich Bi, Ce, Co, Cu, Fe, K, Mg, Ni, P, Pb, Sb, Si, Sn, TI und/oder U ist, und
a gleich 1, b gleich 0,05 bis 1 und c gleich 0 bis 2 ist. Der Gesamtwert von c für Co, Ni und/oder Fe muß dabei weniger als 0,5 betragen. Die Reaktion wird vorzugsweise in Anwesenheit von zugefügtem Wasser durchgeführt. Die offenbarten Katalysatoren können ebenfalls zur Oxidation von Ethan zu Essigsäure verwendet werden, wobei die Effizienz der Umwandlung zu Essigsäure bei ca. 18 %, bei einer Ethan-Umwandlung von 7,5%, liegt.

EP-A-0 294 845 offenbart ein Verfahren zur selektiven Herstellung von Essigsäure aus Ethan, Ethylen oder Gemischen davon mit Sauerstoff in Gegenwart eines Katalysatorgemisches, enthaltend

A.) einen calcinierten Katalysator der Formel $Mo_xV_y$ oder $Mo_xV_yZ_y$, worin Z eines oder mehrere der Metalle Li, Na, Be, Mg, Ca, Sr, Ba, Zn, Cd, Hg, Sc, Y, La, Ce, Al, TI, Ti, Zr, Hf, Pb, Nb, Ta, As, Sb, Bi, Cr, W, U, Te, Fe, Co und Ni sein kann, x gleich 0,5 bis 0,9, y gleich 0,1 bis 0,4 und z gleich 0,001 bis 1 ist, und
B.) einen Ethylenhydratationskatalysator und/oder Ethylenoxidationskatalysator. Bei der zweiten Katalysatorkomponente B handelt es sich insbesondere um einen Molekularsiebkatalysator oder einen Palladium enthaltenden Oxidationskatalysator.

Die erreichbare maximale Selektivität beträgt 27% bei einem Ethanumsatz von 7%.

Die hohen Umsatzraten von Ethan werden gemäß EP-A-0 294 845 nur mit dem beschriebenen Katalysatorgemisch, nicht jedoch mit einem einzigen, die Komponenten A und B enthaltenden Katalysator erreicht.

EP-A-0 407 091 offenbart ein Verfahren zur Herstellung eines Gemischs aus Ethylen und/oder Essigsäure. Hierbei werden Ethan und/oder Ethylen und ein molekularen Sauerstoff enthaltendes Gas bei erhöhter Temperatur mit einer Katalysatorzusammensetzung, die die Elemente A, X und Y enthält, in Kontakt gebracht. A ist hierbei Mo/Re/W, X ist Cr, Mn, Nb, Ta, Ti, V und/oder W und Y ist Bi, Ce, Co, Cu, Fe, K, Mg, Ni, P, Pb, Sb, Si, Sn, Tl und/oder U. Die maximalen Selektivitäten, die bei Verwendung des beschriebenen Katalysators bei der Oxidation von Ethan zu Essigsäure erzielt werden konnten, betragen 78%. Als weitere Nebenprodukte werden Kohlendioxid, Kohlenmonoxid und Ethylen gebildet.

Die genannten Schriften offenbaren Katalysatoren, die Molybdän als Hauptkomponente beinhalten. Katalysatoren, die Molybdän beinhalten sind jedoch nachteilig, da Molybdän unter den herrschenden Reaktionsbedingungen flüchtige Molybdänverbindungen bildet, die zu einer Abnahme der Aktivität und Selektivität des Katalysators führt.

Aus der EP-A-0 620 205 ist ein Verfahren zur einstufigen Umsetzung von Ethylen und Sauerstoff zu Essigsäure bekannt. Der in diesem Dokument beschriebene Katalysator enthält Palladium und Heteropolysäuren bzw. deren Salze. Einer der in diesem Dokument beschriebenen sauerstoffhaltigen Katalysatoren weist neben Palladium und Tellur auch Vanadium, Wolfram und Phosphor auf.

Keine der vorstehend aufgezahlten Publikationen offenbart die Verwendung eines Katalysators, der Wolfram und ein Edelmetall enthält, zur selektiven Oxidation von Ethan zu Essigsäure. Ferner sind die bis jetzt im Stand der Technik erzielten Selektivitäten für diese Oxidation nicht befriedigend.

Es bestand daher die Aufgabe ein Verfahren zur Verfügung zu stellen, mit dem Ethan in einfacher Weise, gezielt und mit hoher Selektivität unter möglichst milden Reaktionsbedingungen zu Essigsäure oxidiert werden kann.

Überraschenderweise wurde gefunden, daß es möglich ist, bei Verwendung eines Katalysators, der Wolfram in Kombination mit einem Edelmetall (z.B. Pd, Pt, Ag und Au) und mehreren Elementen aus der Gruppe Vanadium, Niob, Tantal enthält, Ethan unter relativ milden Bedingungen, in einfacher Weise mit hoher Selektivität zu Essigsäure zu oxidieren. Wolframoxid ist weitaus weniger flüchtig als Molybdänoxid. Somit erweisen sich die erfindungsgemäßen Katalysatoren, die statt Molybdän Wolfram enthalten, über lange Zeit als stabil hinsichtlich ihrer Aktivität und Selektivität.

Die vorliegende Erfindung betrifft somit ein Verfahren zur selektiven Herstellung von Essigsäure aus einer gasförmigen Einspeisung aus Ethan sowie Sauerstoff oder Sauerstoff enthaltenden Gasen bei erhöhter Temperatur an einem Wolfram enthaltenden Katalysator, der die Elemente W, X, Y und Z in den Grammatomverhältnissen a:b:c:d in Kombination mit Sauerstoff enthält

$$W_a X_b Y_c Z_d \hspace{4cm} (I)$$

worin

X   eines oder mehrere Elemente ausgewählt aus der Gruppe Pd, Pt, Ag und/oder Au,

Y   mehrere Elemente ausgewählt aus der Gruppe V, Nb, Cr, Mn, Fe, Sn, Sb, Cu, Zn, U, Ni und/oder Bi,

Z   eines oder mehrere Elemente ausgewählt aus der Gruppe Li, Na, K, Rb, Cs, Be, Mg, Ca, Sr, Ba, Sc, Y, La, Ti, Zr, Hf, Ru, Os, Co, Rh, Ir, B, Al, Ga, In, Tl, Si, Ge, Pb, P, As und/oder Te,

a   1,

b   eine Zahl größer als 0,

c   eine Zahl größer als 0, und

d   eine Zahl von 0 bis 2 ist, mit der Maßgabe, daß der Katalysator als Y mindestens die Elemente V und Nb enthält.

X bedeutet bevorzugt Pd, Y bedeutet bevorzugt V, Nb, Sb und/oder Cu, und Z bedeutet bevorzugt K, Ca, Si und/oder P.

Sofern X, Y und Z für mehrere verschiedene Elemente stehen, können die Indizes b, c und d ebenfalls mehrere unterschiedliche Werte annehmen.

Weiterhin betrifft die vorliegende Erfindung einen Katalysator zur selektiven Herstellung von Essigsäure enthaltend die Elemente W, X, Y und Z in den Grammatomverhältnissen a:b:c:d in Kombination mit Sauerstoff.

Vorzugswesie bedeuten die stöchiometrischen Indizes b, c und d:

b   0,0001 bis 0,5;

c   0,1 bis 1,0, und

d   0,001 bis 1,0.

Werte von b, die oberhalb des Vorzugsbereichs liegen, können bei dem erfindungsgemäßen Verfahren zu einer Begünstigung der Kohlendioxidbildung führen. Dagegen wird bei Gehalten unterhalb des angegebenen Vorzugsbereichs eine Bevorzugung der Ethylenbildung beobachtet. Weiterhin ermöglichen die bevorzugten Werte für b eine besonders wirtschaftliche Ausführung der Erfindung.

In einer weiteren bevorzugten Ausführungsform enthält der erfindungsgemäße Katalysator außer den Elementen Wolfram, Palladium, Vanadium, Niob noch Antimon und Calcium in Kombination mit Sauerstoff. Die Grammatomverhältnisse $a:b:c^1:c^2:c^3:d^1$ der Elemente W:Pd:V:Nb:Sb:Ca sind vorzugsweise wie folgt:

a (W) = 1;

b (Pd) = 0,0001 bis 0,5, insbesondere 0,0002 bis 0,05;

$c^1$ (V) = 0,1 bis 1,0;

$c^2$ (Nb) = 0,1 bis 0,5;

$c^3$ (Sb) = 0 bis 0,5;

$d^1$ (Ca) = 0 bis 0,2.

Beispiele für im erfindungsgemäßen Verfahren besonders bevorzugt eingesetzte Katalysatoren sind.

$W_{1,00}Pd_{0,0005}V_{0,50}Nb_{0,12}$

$W_{1,00}Pd_{0,0005}V_{0,75}Nb_{0,20}$

$W_{1,00}Pd_{0,0004}V_{0,50}Nb_{0,20}Cu_{0,10}P_{0,05}$

$W_{1,00}Pd_{0,0005}V_{0,50}Nb_{0,12}Sb_{0,10}Ca_{0,02}$

$W_{1,00}Pd_{0,0004}Au_{0,0001}V_{0,75}Nb_{0,25}Te_{0,002}$

$W_{1,00}Pd_{0,0005}Ag_{0,0001}V_{0,75}Nb_{0,12}Si_{0,01}$

Die erfindungsgemäßen Katalysatoren können nach den im Stand der Technik beschriebenen Verfahren hergestellt werden. Hierzu geht man von einer Aufschlämmung, insbesondere einer wäßrigen Lösung, die die einzelnen Ausgangskomponenten der Elemente entsprechend ihrer Anteile enthält, aus.

Die Ausgangsmaterialien der Einzelkomponenten zur Herstellung des erfindungsgemäßen Katalysators sind neben den Oxiden vorzugsweise in Wasser lösliche Substanzen wie Ammoniumsalze, Nitrate, Sulfate, Halogenide, Hydroxide und Salze organischer Säuren, die durch Erwärmung in die entsprechenden Oxide umgewandelt werden können. Zur Vermischung der Komponenten werden wäßrige Lösungen oder Suspensionen der Metallverbindungen hergestellt und vermischt.

Bei Wolfram empfiehlt es sich aufgrund der kommerziellen Verfügbarkeit als Ausgangsmaterialien die entsprechenden Wolframate, wie z.B. Ammoniumwolframat, einzusetzen.

Die erhaltene Reaktionsmischung wird dann 5 Minuten bis 5 Stunden bei 50 bis 100 °C gerührt. Anschließend wird das Wasser entfernt und der verbleibende Katalysator bei einer Temperatur von 50 bis 150°C, insbesondere 80 bis 120°C getrocknet.

Für den Fall, daß der erhaltene Katalysator anschließend noch einem Kalzinierungsprozeß unterworfen wird, empfiehlt es sich den getrockneten und pulverisierten Katalysator bei einer Temperatur im Bereich von 100°C bis 800°C, insbesondere 200 bis 500°C in Gegenwart von Stickstoff, Sauerstoff oder eines sauerstoffhaltigen Gases zu kalzinieren. Die Zeitdauer für die Kazinierung beträgt vorzugsweise 2 bis 24 Stunden.

Der Katalysator kann ohne ein entsprechendes Trägermaterial eingesetzt werden oder mit einem solchen gemischt oder auf ein solches aufgebracht werden. Geeignet sind übliche Trägermaterialien, wie z.B. poröses Siliziumdioxid, geglühtes Siliziumdioxid, Kieselgur, Kieselgel, poröses oder nicht poröses Aluminiumoxid, Titandioxid, Zirkoniumdioxid, Thoriumdioxid, Lanthanoxid, Magnesiumoxid, Calciumoxid, Bariumoxid, Zinnoxid, Cerdioxid, Zinkoxid, Boroxid, Bornitrid, Borcarbid, Borphosphat, Zirkoniumphosphat, Aluminiumsilikat, Siliziumnitrid oder Siliziumcarbid aber auch Glas-, Kohlefaser, Metalloxid,- oder Metallnetze oder entsprechende Monolithe.

Wird der Katalysator auf einen Träger aufgebracht, so kann dies durch trockene oder feuchte Imprägnierung des Trägers mit den gelösten oder suspendierten Komponenten des Katalysators erfolgen. Eine andere Möglichkeit ist, die Lösungen oder Suspensionen der Katalysatorkomponente mit einem Sol des Trägermaterials zu mischen und anschließend einer Sprühtrocknung zu unterziehen. In beiden Fällen kann anschließend wie beschrieben kalziniert werden.

Bevorzugte Trägermaterialien haben eine Oberfläche von weniger als 100 m$^2$/g. Bevorzugte Trägermaterialien sind Siliziumdioxid und Aluminiumoxid mit geringer spezifischer Oberfläche. Der Katalysator kann nach der Formgebung als regelmäßig oder unregelmäßig geformter Trägerkörper, in Pulverform oder in den oben genannten Formen als heterogener Oxidationskatalysator eingesetzt werden.

Die Reaktion kann in der Wirbelschicht oder in einem Festbettreaktor durchgeführt werden. Für den Einsatz in einer Wirbelschicht wird der Katalysator üblicherweise auf eine Korngröße im Bereich von 10 bis 200 μm gemahlen oder durch Sprühtrocknung hergestellt.

Die gasförmige Einspeisung enthält Ethan, welches als reines Gas oder in Mischung mit einem oder mehreren anderen Gasen dem Reaktor zugeführt wird. Als solche zusätzlichen oder Trägergase kommen beispielsweise Stickstoff, Methan, Kohlenmonoxid, Kohlendioxid, Luft und/oder Wasserdampf in Frage. Das molekularen Sauerstoff enthaltende Gas kann Luft oder ein an molekularen Sauerstoff reicheres oder ärmeres Gas als Luft, z.B. reiner Sauerstoff, sein. Der Anteil des Wasserdampfes kann im Bereich von 0 bis 50 Vol% liegen. Höhere Wasserdampfkonzentrationen würden die Aufarbeitung der anfallenden wässrigen Essigsäure aus verfahrenstechnischen Gründen unnötig verteuern, sind aber technisch möglich. Das molare Verhältnis von Ethan zu Sauerstoff liegt bevorzugt im Bereich zwischen 1:1 und 10:1, insbesondere 2:1 und 8:1. Höhere Sauerstoffgehalte sind bevorzugt, da der erreichbare Ethanumsatz und somit die Ausbeute an Essigsäure höher ist. Bevorzugt ist die Zugabe von Sauerstoff oder des molekularen Sauerstoff enthaltenden Gases in einem Konzentrationsbereich außerhalb der Explosionsgrenzen unter Reaktionsbedingungen, da hierdurch die Durchführung des Verfahrens vereinfacht wird. Allerdings ist es auch möglich das Ethan/Sauerstoff-Gemisch innerhalb der Explosionsgrenzen einzustellen.

Die Raktion wird im allgemeinen bei Temperaturen zwischen 200 und 500°C, bevorzugt 200 bis 400°C durchgeführt. Der Druck kann atmosphärisch oder superatmosphärisch sein, z.B. im Bereich zwischen 1 und 50 bar, bevorzugt 1 bis 30 bar.

Die Reaktion kann in einem Festbett- oder Wirbelschichtreaktor durchgeführt werden. Zweckmäßigerweise wird Ethan zunächst mit den inerten Gasen wie Stickstoff oder Wasserdampf gemischt, bevor Sauerstoff oder das molekularen Sauerstoff enthaltende Gas zugeführt wird. Die vermischten Gase werden bevorzugt in einer Vorheizzone auf die Reaktionstemperatur vorgeheizt, bevor das Gasgemisch mit dem Katalysator in Kontakt gebracht wird. Aus dem Reaktorabgas wird Essigsäure durch Kondensation abgetrennt. Die übrigen Gase werden an den Reaktoreingang zurückgeführt, wo Sauerstoff oder das molekularen Sauerstoff enthaltende Gas sowie Ethan zudosiert wird.

Beispiele

Die in den Beispielen aufgeführte Katalysatorzusammensetzung ist in relativen Atomverhältnissen angegeben.

Katalysatorpräparation:

Katalysator (I):

Ein Katalysator mit folgender Zusammensetzung wurde hergestellt:
$W_{1,00}Pd_{0,0005}V_{0,25}Nb_{0,12}$
100 g Ammonium-meta-wolframat werden in 500 ml Wasser bei 90°C suspendiert. Zu dieser Mischung wird eine Lösung aus 11,2 g Ammonium-meta-vanadat in 250 ml 90°C warmen Wasser getropft. Die vereinten Mischungen werden bei 90°C für 15 Minuten gerührt. Anschließend tropft man zu dieser Mischung eine Lösung aus 35,7 g Nioboxalat in 400 ml 90°C warmen Wasser. Die vereinten Lösungen werden bei 90°C für 15 Minuten gerührt. Zuletzt fügt man zu der erhaltenen Mischung eine Lösung aus 0,043 g Palladiumacetat in 50 ml Aceton hinzu und rührt die Mischung bei 90°C für 15 Minuten. Danach wird das Wasser verdampft und der eingedampfte Rückstand bei 120°C über Nacht getrocknet. Der Feststoff wird zerstoßen (Siebfraktion < 2 mm) und anschließend unter einem Luftstrom auf 400°C erhitzt mit einer Aufheizrate von 2°C pro Minute. Die Temperatur wird für vier Stunden gehalten. Der Luftstrom wird abgestellt und das Material langsam abgekühlt. Der Katalysator wird gemörsert und gepreßt (Preßdruck 2 Tonnen) und gesiebt, um eine Siebfraktion zwischen 0,35 und 0,7 mm zu erhalten.

Katalysator (II):

$W_{1,00}Pd_{0,0005}V_{0,50}Nb_{0,12}$
100 g Ammonlum-meta-wolframat werden in 500 ml Wasser bei 90°C suspendiert. Zu dieser Mischung wird eine Lösung aus 22,4 g Ammonium-meta-vanadat in 250 ml 90°C warmen Wasser getropft. Die vereinten Mischungen werden bei 90°C für 15 Minuten gerührt. Anschließend tropft man zu dieser Mischung eine Lösung aus 35,7 g Nioboxalat in 400 ml 90°C warmen Wasser. Die vereinten Lösungen werden bei 90°C für 15 Minuten gerührt. Zuletzt fügt man zu der erhaltenen Mischung eine Lösung aus 0,043 g Palladiumacetat in 50 ml Aceton hinzu und rührt die Mischung bei 90°C für 15 Minuten. Danach wird das Wasser verdampft und der eingedampfte Rückstand bei 120°C über Nacht getrocknet. Der Feststoff wird zerstoßen (Siebfraktion < 2 mm) und anschließend unter einem Luftstrom auf 400°C erhitzt mit einer Aufheizrate von 2°C pro Minute. Die Temperatur wird für vier Stunden gehalten. Der Luftstrom wird abgestellt und das Material langsam abgekühlt. Der Katalysator wird gemörsert und gepreßt (Preßdruck 2 Tonnen) und gesiebt, um eine Siebfraktion zwischen 0,35 und 0,7 mm zu erhalten.

Katalysator (III):

$W_{1,00}Pd_{0,0005}V_{1,00}Nb_{0,12}$
100 g Ammonium meta wolframat werden in 500 ml Wasser bei 90°C suspendiert. Zu dieser Mischung wird eine Lösung aus 44,4 g Ammonium-meta-vanadat in 250 ml 90°C warmen Wasser getropft. Die vereinten Mischungen werden bei 90°C für 15 Minuten gerührt. Anschließend tropft man zu dieser Mischung eine Lösung aus 35,7 g Nioboxalat in 400 ml 90°C warmen Wasser. Die vereinten Lösungen werden bei 90°C für 15 Minuten gerührt. Zuletzt fügt man zu der erhaltenen Mischung eine Lösung aus 0,043 g Palladiumacetat in 50 ml Aceton hinzu und rührt die Mischung bei 90°C für 15 Minuten. Danach wird das Wasser verdampft und der eingedampfte Rückstand bei 120°C über Nacht getrocknet. Der Feststoff wird zerstoßen (Siebfraktion < 2 mm) und anschließend unter einem Luftstrom auf 400°C erhitzt mit einer Aufheizrate von 2°C pro Minute. Die Temperatur wird für vier Stunden gehalten. Der Luftstrom wird abgestellt und das Material langsam abgekühlt. Der Katalysator wird gemörsert und gepreßt (Preßdruck 2 Tonnen) und gesiebt, um eine Siebfraktion zwischen 0,35 und 0,7 mm zu erhalten.

Katalysator (IV):

$W_{1,00}Pd_{0,0005}V_{0,50}Nb_{0,12}Sb_{0,10}Ca_{0,02}$
100 g Ammonium-meta-wolframat werden in 500 ml Wasser bei 90°C suspendiert. Zu dieser Mischung wird eine Lösung aus 22,4 g Ammonium-meta-vanadat in 250 ml 90°C warmen Wasser getropft. Die vereinten Mischungen werden bei 90°C für 15 Minuten gerührt. Anschließend tropft man zu dieser Mischung eine Lösung aus 35,7 g Nioboxalat, 9,7 g Antimonoxalat und 1,8 g Calciumnitrat in 400 ml 90°C warmen Wasser. Die vereinten Lösungen werden bei 90°C für 15 Minuten gerührt. Zuletzt fügt man zu der erhaltenen Mischung eine Lösung aus 0,043 g Palladiumacetat in 50 ml Aceton hinzu und rührt die Mischung bei 90°C für 15 Minuten. Danach wird das Wasser verdampft und der eingedampfte Rückstand bei 120°C über Nacht getrocknet. Der Feststoff wird zerstoßen (Siebfraktion < 2 mm) und anschließend unter einem Luftstrom auf 400°C erhitzt mit einer Aufheizrate von 2°C pro Minute. Die Temperatur wird für vier Stunden gehalten. Der Luftstrom wird abgestellt und das Material langsam abgekühlt. Der Katalysator wird gemörsert und gepreßt (Preßdruck 2 Tonnen) und gesiebt, um eine Siebfraktion zwischen 0,35 und 0,7 mm zu erhalten.

Katalysator (V):

$W_{1,00}Pd_{0,0005}Ag_{0,0001}V_{0,75}Nb_{0,12}Si_{0,01}$

100 g Ammonium-meta-wolframat werden in 500 ml Wasser bei 90°C suspendiert. Zu dieser Mischung wird eine Lösung aus 33,6 g Ammonium-meta-vanadat in 250 ml 90°C warmen Wasser getropft. Die vereinten Mischungen werden bei 90°C für 15 Minuten gerührt. Anschließend tropft man zu dieser Mischung eine Suspension aus 35,7 g Nioboxalat, 0,01 g Silbernitrat und 0,23 g Kieselgur in 400 ml 90°C warmen Wasser. Die vereinten Lösungen werden bei 90°C für 15 Minuten gerührt. Zuletzt fügt man zu der erhaltenen Mischung eine Lösung aus 0,043 g Palladiumacetat in 50 ml Aceton hinzu und rührt die Mischung bei 90°C für 15 Minuten. Danach wird das Wasser verdampft und der eingedampfte Rückstand bei 120°C über Nacht getrocknet. Der Feststoff wird zerstoßen (Siebfraktion < 2 mm) und anschließend unter einem Luftstrom auf 400°C erhitzt mit einer Aufheizrate von 2°C pro Minute. Die Temperatur wird für vier Stunden gehalten. Der Luftstrom wird abgestellt und das Material langsam abgekühlt. Der Katalysator wird gemörsert und gepreßt (Preßdruck 2 Tonnen) und gesiebt, um eine Siebfraktion zwischen 0,35 und 0,7 mm zu erhalten.

Katalysator (VI):

$W_{1,00}Pd_{0,0004}V_{0,50}Nb_{0,2}Cu_{0,10}P_{0,05}$

100 g Ammonium-meta-wolframat werden in 500 ml Wasser bei 90°C suspendiert. Zu dieser Mischung wird eine Lösung aus 22,4 g Ammonium-meta-vanadat in 250 ml 90°C warmen Wasser getropft. Die vereinten Mischungen werden bei 90°C für 15 Minuten gerührt. Anschließend tropft man zu dieser Mischung eine Suspension aus 59,5 g Nioboxalat, 8,91 g Kupfernitrat und 1,6 g Phosphorsäure (85%) in 400 ml 90°C warmen Wasser. Die vereinten Lösungen werden bei 90°C für 15 Minuten gerührt. Zuletzt fügt man zu der erhaltenen Mischung eine Lösung aus 0,034 g Palladiumacetat in 50 ml Aceton hinzu und rührt die Mischung bei 90°C für 15 Minuten. Danach wird das Wasser verdampft und der eingedampfte Rückstand bei 120°C über Nacht getrocknet. Der Feststoff wird zerstoßen (Siebfraktion < 2 mm) und anschließend unter einem Luftstrom auf 400°C erhitzt mit einer Aufheizrate von 2°C pro Minute. Die Temperatur wird für vier Stunden gehalten. Der Luftstrom wird abgestellt und das Material langsam abgekühlt. Der Katalysator wird gemörsert und gepreßt (Preßdruck 2 Tonnen) und gesiebt, um eine Siebfraktion zwischen 0,35 und 0,7 mm zu erhalten.

Katalysator (VII):

$W_{1,00}Pd_{0,0003}Au_{0,0001}V_{0,75}Nb_{0,25}Te_{0,002}$

100 g Ammonium-meta-wolframat werden in 500 ml Wasser bei 90°C suspendiert. Zu dieser Mischung wird eine Lösung aus 22,4 g Ammonium-meta-vanadat in 250 ml 90°C warmen Wasser getropft. Die vereinten Mischungen werden bei 90°C für 15 Minuten gerührt. Anschließend tropft man zu dieser Mischung eine Lösung aus 74,4 g Nioboxalat, 0,015 g Tetrachlorogoldsäure und 0,18 g Tellursäure in 400 ml 90°C warmen Wasser. Die vereinten Lösungen werden bei 90°C für 15 Minuten gerührt. Zuletzt fügt man zu der erhaltenen Mischung eine Lösung aus 0,026 g Palladiumacetat in 50 ml Aceton hinzu und rührt die Mischung bei 90°C für 15 Minuten. Danach wird das Wasser verdampft und der eingedampfte Rückstand bei 120°C über Nacht getrocknet. Der Feststoff wird zerstoßen (Siebfraktion < 2 mm) und anschließend unter einem Luftstrom auf 400°C erhitzt mit einer Aufheizrate von 2°C pro Minute. Die Temperatur wird für vier Stunden gehalten. Der Luftstrom wird abgestellt und das Material langsam abgekühlt. Der Katalysator wird gemörsert und gepreßt (Preßdruck 2 Tonnen) und gesiebt, um eine Siebfraktion zwischen 0,35 und 0,7 mm zu erhalten.

Verfahren zur Katalysatoraustestung

10 ml des Katalysators wurden in einen Stahlreaktor mit 10 mm Innendurchmesser geladen. Der Katalysator wurde unter einem Luftstrom auf 250°C aufgeheizt. Anschließend wurde der Druck mittels eines Vordruckreglers eingestellt. Das gewünschte Ethan/Sauerstoff/Stickstoff-Gemisch wurde mit Wasser in eine Verdampferzone eindosiert, wo Wasser verdampfte und mit den Gasen vermischt wurde. Die Reaktionstemperatur wurde mit einem Thermoelement in der Katalysatorschüttung gemessen. Das Reaktionsgas wurde On-Line gaschromatographisch analysiert.

In den Beispielen sind die folgende Begriffe definiert als:

$$\text{Ethanumsatz (\%)} =$$

$$100 \times ([CO]/2+[CO_2]/2+[C_2H_4]+[CH_3COOH])/([CO]/_2+[CO_2]/2+[C_2H_4]+[C_2H_6] +$$

$$[CH_3COOH])$$

$$Ethylenselektivität\ (\%) =$$

$$100x([C_2H_4])/([CO]/_2+[CO_2]/2+[C_2H_4]+[CH_3COOH])$$

$$Essigsäureselektiviät\ (\%) =$$

$$100x([CH_3COOH])/([CO]/_2+[CO_2]/2+[C_2H_4]+[CH_3COOH])$$

worin

[ ] = Konzentrationen in Mol% und
$[C_2H_6]$ = Konzentration des nicht umgesetzten Ethans bedeutet.

Die Verweilzeit ist definiert als:
t (s) = Schüttvolumen des Katalysators (ml) / Volumenstrom des Gases durch den Reaktor bezogen auf die Reaktionsbedingungen (ml/s)

Reaktionsdurchführung:

Das Reaktoreingangsgas bestand aus 40 Vol% Ethan, 8 Vol% Sauerstoff, 32 Vol% Stickstoff und 20 Vol% Wasserdampf. Die Reaktionsbedingungen und Ergebnisse sind in nachfolgender Tabelle zusammengefaßt.

| Bsp. | Katalysator | Temperatur (°C) | Druck (bar) | Verweilzeit (s) | Ethanumsatz (%) | Essigsäureselektivität (%) | Ethylenselektivität (%) | CO + $CO_2$ Selektivität (%) |
|---|---|---|---|---|---|---|---|---|
| 1 | I | 280 | 15 | 30 | 7 | 57 | 2 | 41 |
| 2 | I | 290 | 15 | 30 | 8 | 56 | 3 | 41 |
| 3 | I | 300 | 15 | 30 | 9 | 56 | 3 | 41 |
| 4 | II | 260 | 15 | 30 | 8 | 76 | 1 | 23 |
| 5 | II | 280 | 15 | 30 | 10 | 74 | 2 | 24 |
| 6 | II | 280 | 30 | 15 | 10 | 75 | 3 | 22 |
| 7 | III | 260 | 15 | 30 | 9 | 81 | 0 | 19 |
| 8 | III | 270 | 15 | 30 | 11 | 79 | 0 | 21 |
| 9 | III | 280 | 15 | 30 | 11 | 78 | 1 | 21 |
| 10 | IV | 260 | 15 | 30 | 7 | 80 | 1 | 19 |
| 11 | IV | 280 | 15 | 30 | 10 | 75 | 4 | 21 |
| 12 | V | 250 | 15 | 25 | 8 | 78 | 1 | 21 |
| 13 | V | 260 | 15 | 20 | 9 | 77 | 3 | 20 |
| 14 | VI | 250 | 15 | 20 | 10 | 80 | 1 | 19 |
| 15 | VII | 280 | 15 | 30 | 8 | 77 | 2 | 21 |

**Patentansprüche**

1. Verfahren zur selektiven Herstellung von Essigsäure aus einer gasförmige Einspeisung aus Ethan sowie Sauerstoff oder Sauerstoff enthaltenden Gasen bei erhöhter Temperatur an einem Wolfram enthaltenden Katalysator, der die Elemente W, X, Y und Z in den Grammatomverhältnissen a:b:c:d in Kombination mit Sauerstoff enthält

$$W_aX_bY_cZ_d \qquad (I),$$

worin

    x    eines oder mehrere Elemente ausgewählt aus der Gruppe Pd, Pt, Ag und/oder Au,
    Y    mehrere Elemente ausgewählt aus der Gruppe V, Nb, Cr, Mn, Fe, Sn, Sb, Cu, Zn, U, Ni und/oder Bi,
    Z    eines oder mehrere Elemente ausgewählt aus der Gruppe Li, Na, K, Rb, Cs, Be, Mg, Ca, Sr, Ba, Sc, Y, La, Ti, Zr, Hf, Ru, Os, Co, Rh, Ir, B, Al, Ga, In, Tl, Si, Ge, Pb, P, As und/oder Te,
    a    1,
    b    eine Zahl größer als 0,
    c    eine Zahl größer als 0, und
    d    eine Zahl von 0 bis 2 ist, mit der Maßgabe, daß der Katalysator als Y mindestens die Elemente V und Nb enthält.

2. Verfahren nach Anspruch 1, **dadurch gekennzeichnet, daß** X, Y und/oder Z jeweils für mehrere Elemente stehen, wobei die Indizes b, c und d für verschiedene Elemente unterschiedliche Werte annehmen können.

3. Verfahren nach mindestens einem der Ansprüche 1 bis 2, **dadurch gekennzeichnet, daß** die Reaktionstemperatur 200 bis 500°C, bevorzugt 200 bis 400°C beträgt.

4. Verfahren nach mindestens einem der Ansprüche 1 bis 3, **dadurch gekennzeichnet, daß** der Druck im Reaktor zwischen 1 und 50 bar, bevorzugt zwischen 1 und 30 bar beträgt.

5. Verfahren nach mindestens einem der Ansprüche 1 bis 4, **dadurch gekennzeichnet, daß** b eine Zahl von 0,0001 bis 0,5 bedeutet.

6. Verfahren nach mindestens einem der Ansprüche 1 bis 5, **dadurch gekennzeichnet, daß** c eine Zahl von 0,1 bis 1,0 bedeutet.

7. Verfahren nach mindestens einem der Ansprüche 1 bis 6, **dadurch gekennzeichnet, daß** d eine Zahl von 0 bis 1,0 bedeutet.

8. Verfahren nach mindestens einem der Ansprüche 1 bis 7, **dadurch gekennzeichnet, daß** dem Reaktor Ethan gemischt mit mindestens einem weiteren Gas zugeführt wird.

9. Verfahren nach Anspruch 8, **dadurch gekennzeichnet, daß** als weiteres Gas Stickstoff, Sauerstoff, Methan, Kohlenmonoxid, Kohlendioxid und/oder Wasserdampf zugeführt wird.

10. Verfahren nach mindestens einem der Ansprüche 1 bis 9, **dadurch gekennzeichnet, daß** der Katalysator mindestens eine der folgenden Zusammensetzungen in Kombination mit Sauerstoff enthält:

    $W_{1,00}Pd_{0,0005}V_{0,50}Nb_{0,12}$
    $W_{1,00}Pd_{0,0005}V_{0,75}Nb_{0,20}$
    $W_{1,00}Pd_{0,0004}V_{0,50}Nb_{0,20}Cu_{0,10}P_{0,05}$
    $W_{1,00}Pd_{0,0005}V_{0,50}Nb_{0,12}Sb_{0,10}Ca_{0,02}$
    $W_{1,00}Pd_{0,0004}Au_{0,0001}V_{0,75}Nb_{0,25}Te_{0,002}$
    $W_{1,00}Pd_{0,0005}Ag_{0,0001}V_{0,75}Nb_{0,12}Si_{0,01}$

11. Verfahren nach mindestens einem der Ansprüche 1 bis 10, **dadurch gekennzeichnet, daß** der Katalysator mit einem Trägermaterial gemischt oder auf einem Trägermaterial fixiert ist.

**12.** Verfahren nach mindestens einem der Ansprüche 1 bis 11, **dadurch gekennzeichnet, daß** das molare Verhältnis von Ethan zu Sauerstoff zwischen 1:1 und 10:1, vorzugsweise zwischen 2:1 und 8:1 liegt.

**13.** Wolframhaltiger Katalysator zur selektiven Herstellung von Essigsäure aus Ethan sowie Sauerstoff, enthaltend die Elemente W, X, Y und Z in den Grammatomverhältnissen a:b:c:d in Kombination mit Sauerstoff

$$W_aX_bY_cZ_d \qquad \text{(I)}$$

worin

x   eines oder mehrere Elemente ausgewählt aus der Gruppe Pd, Pt, Ag und/oder Au,
Y   mehrere Elemente ausgewählt aus der Gruppe V, Nb, Cr, Mn, Fe, Sn, Sb, Cu, Zn, U, Ni und/oder Bi,
Z   eines oder mehrere Elemente ausgewählt aus der Gruppe Li, Na, K, Rb, Cs, Be, Mg, Ca, Sr, Ba, Sc, Y, La, Ti, Zr, Hf, Ru, Os, Co, Rh, Ir, B, Al, Ga, In, Tl, Si, Ge, Pb, P, As und/oder Te,
a   1,
b   eine Zahl größer als 0,
c   eine Zahl größer als 0, und
d   eine Zahl von 0 bis 2 ist, mit der Maßgabe, daß der Katalysator als Y mindestens die Elemente V und Nb enthält.

**Claims**

**1.** A process for the selective preparation of acetic acid from a gaseous feedstock of ethane, and oxygen or oxygen-containing gases, at elevated temperature on a tungsten-containing catalyst which comprises the elements W, X, Y and Z in the gram-atom ratios a:b:c:d in combination with oxygen

$$W_aX_bY_cZ_d \qquad \text{(I)}$$

in which

x   is one or more elements selected from the group of Pd, Pt, Ag and/or Au,
Y   is a plurality of elements selected from the group of V, Nb, Cr, Mn, Fe, Sn, Sb, Cu, Zn, U, Ni and/or Bi,
Z   is one or more elements selected from the group of Li, Na, K, Rb, Cs, Be, Mg, Ca, Sr, Ba, Sc, Y, La, Ti, Zr, Hf, Ru, Os, Co, Rh, Ir, B, Al, Ga, In, Tl, Si, Ge, Pb, P, As and/or Te,
a   is 1,
b   is a number greater than 0,
c   is a number greater than 0, and
d   is a number from 0 to 2, with the proviso that the catalyst contains, as Y, at least the elements V and Nb.

**2.** The process as claimed in claim 1, wherein X, Y and/or Z are each several elements, where the indices b, c and d can assume different values for different elements.

**3.** The process as claimed in at least one of claims 1 to 2, wherein the reaction temperature is 200 to 500°C, preferably 200 to 400°C.

**4.** The process as claimed in at least one of claims 1 to 3, wherein the pressure in the reactor is between 1 and 50 bar, preferably between 1 and 30 bar.

**5.** The process as claimed in at least one of claims 1 to 4, wherein b is a number from 0.0001 to 0.5.

**6.** The process as claimed in at least one of claims 1 to 5, wherein c is a number from 0.1 to 1.0.

**7.** The process as claimed in at least one of claims 1 to 6, wherein d is a number from 0 to 1.0.

8. The process as claimed in at least one of claims 1 to 7, wherein ethane mixed with at least one other gas is fed into the reactor.

9. The process as claimed in claim 8, wherein the other gas fed in is nitrogen, oxygen, methane, carbon monoxide, carbon dioxide, and/or steam.

10. The process as claimed in at least one of claims 1 to 9, wherein the catalyst comprises at least one of the following compositions in combination with oxygen:

$W_{1,00}Pd_{0,0005}V_{0,50}Nb_{0,12}$
$W_{1,00}Pd_{0,0005}V_{0,75}Nb_{0,20}$
$W_{1,00}Pd_{0,0004}V_{0,50}Nb_{0,20}Cu_{0,10}P_{0,05}$
$W_{1,00}Pd_{0,0005}V_{0,50}Nb_{0,12}Sb_{0,10}Ca_{0,02}$
$W_{1,00}Pd_{0,0004}Au_{0,0001}V_{0,75}Nb_{0,25}Te_{0,002}$
$W_{1,00}Pd_{0,0005}Ag_{0,0001}V_{0,75}Nb_{0,12}Si_{0,01}$

11. The process as claimed in at least one of claims 1 to 10, wherein the catalyst is mixed with a carrier material or is immobilized on a carrier material.

12. The process as claimed in at least one of claims 1 to 11, wherein the molar ratio of ethane to oxygen is between 1:1 and 10:1, preferably between 2:1 and 8:1.

13. A tungsten-containing catalyst for the selective preparation of acetic acid from ethane, and oxygen, comprising the elements W, X, Y and Z in the gram-atom ratios a:b:c:d in combination with oxygen

$$W_aX_bY_cZ_d \tag{I}$$

in which

x     is one or more elements selected from the group of Pd, Pt, Ag and/or Au,
Y     is a plurality of elements selected from the group of V, Nb, Cr, Mn, Fe, Sn, Sb, Cu, Zn, U, Ni and/or Bi,
Z     is one or more elements selected from the group of Li, Na, K, Rb, Cs, Be, Mg, Ca, Sr, Ba, Sc, Y, La, Ti, Zr, Hf, Ru, Os, Co, Rh, Ir, B, Al, Ga, In, Tl, Si, Ge, Pb, P, As and/or Te,
a     is 1,
b     is a number greater than 0,
c     is a number greater than 0, and
d     is a number from 0 to 2, with the proviso that the catalyst contains, as Y, at least the elements V and Nb.

**Revendications**

1. Procédé pour la préparation sélective d'acide acétique à partir d'une alimentation gazeuse d'éthane ainsi que d'oxygène ou de gaz contenant de l'oxygène à une température élevée sur un catalyseur contenant du tungstène, lequel contient les éléments W, X, Y et Z dans les rapports d'atome-gramme a:b:c:d en combinaison avec de l'oxygène

$$W_aX_bY_cZ_d \tag{I}$$

où

x     est un ou plusieurs éléments choisis parmi Pd, Pt, Ag et/ou Au,
Y     représente plusieurs éléments choisis parmi V, Nb, Cr, Mn, Fe, Sn, Sb, Cu, Zn, U, Ni et/ou Bi,
Z     représente un ou plusieurs éléments choisis parmi Li, Na, K, Rb, Cs, Be, Mg, Ca, Sr, Ba, Sc, Y, La, Ti, Zr, Hf, Ru, Os, Co, Rh, Ir, B, Al, Ga, In, Tl, Si, Ge, Pb, P, As et/ou Te,
a     est égal à 1,

b   est un nombre supérieur à 0,

c   est un nombre supérieur à 0, et

d   est un nombre compris entre 0 et 2, à condition que le catalyseur contienne comme Y au moins les éléments V et Nb.

2.  Procédé selon la revendication 1, **caractérisé en ce que** X, Y et/ou Z représentent à chaque fois plusieurs éléments, les indices b, c et d pouvant prendre pour des éléments différents des valeurs différentes.

3.  Procédé selon au moins l'une quelconque des revendications 1 à 2, **caractérisé en ce que** la température de la réaction est comprise entre 200 et 500°C, de préférence entre 200 et 400°C.

4.  Procédé selon au moins l'une quelconque des revendications 1 à 3, **caractérisé en ce que** la pression dans le réacteur est comprise entre 1 et 50 bar, de préférence entre 1 et 30 bar.

5.  Procédé selon au moins l'une quelconque des revendications 1 à 4, **caractérisé en ce que** b indique un nombre compris entre 0,0001 et 0,5.

6.  Procédé selon au moins l'une quelconque des revendications 1 à 5, **caractérisé en ce que** c est un nombre compris entre 0,1 et 1,0.

7.  Procédé selon au moins l'une quelconque des revendications 1 à 6, **caractérisé en ce que** d est un nombre compris entre 0 et 1,0.

8.  Procédé selon au moins l'une quelconque des revendications 1 à 7, **caractérisé en ce que** l'on introduit dans le réacteur de l'éthane mélangé avec au moins un autre gaz.

9.  Procédé selon la revendication 8, **caractérisé en ce que** l'on introduit comme autre gaz de l'azote, de l'oxygène, du méthane, du monoxyde de carbone, du dioxyde de carbone et/ou de la vapeur d'eau.

10. Procédé selon au moins l'une quelconque des revendications 1 à 9, **caractérisé en ce que** le catalyseur contient au moins une des compositions suivantes en combinaison avec de l'oxygène :

$W_{1,00}Pd_{0,0005}V_{0,50}Nb_{0,12}$
$W_{1,00}Pd_{0,0005}V_{0,75}Nb_{0,20}$
$W_{1,00}Pd_{0,0004}V_{0,50}Nb_{0,20}Cu_{0,10}P_{0,05}$
$W_{1,00}Pd_{0,0005}V_{0,50}Nb_{0,12}Sb_{0,10}Ca_{0,02}$
$W_{1,00}Pd_{0,0004}Au_{0,0001}V_{0,75}Nb_{0,25}Te_{0,002}$
$W_{1,00}Pd_{0,0005}Ag_{0,0001}V_{0,75}Nb_{0,12}Si_{0,01}$

11. Procédé selon au moins l'une quelconque des revendications 1 à 10, **caractérisé en ce que** le catalyseur est mélangé avec une matière de support ou est fixé sur une matière de support.

12. Procédé selon au moins l'une quelconque des revendications 1 à 11, **caractérisé en ce que** le rapport molaire de l'éthane à l'oxygène est compris entre 1:1 et 10:1, de préférence entre 2:1 et 8:1.

13. Catalyseur contenant du tungstène pour la préparation sélective d'acide acétique à partir d'éthane ainsi que d'oxygène, contenant les éléments W, X, Y et Z dans les rapports d'atome-gramme a:b:c:d en combinaison avec de l'oxygène

$$W_aX_bY_cZ_d \tag{I}$$

où

x   représente un ou plusieurs éléments choisis parmi Pd, Pt, Ag et/ou Au,

Y   représente plusieurs éléments choisis parmi V, Nb, Cr, Mn, Fe, Sn, Sb, Cu, Zn, U, Ni et/ou Bi,

z   représente un ou plusieurs éléments choisis parmi Li, Na, K, Rb, Cs, Be, Mg, Ca, Sr, Ba, Sc, Y, La, Ti, Zr, Hf, Ru, Os, Co, Rh, Ir, B, Al, Ga, In, Tl, Si, Ge, Pb, P, As et/ou Te,

a     est égal à 1,

b     est un nombre supérieur à 0,

c     est un nombre supérieur à 0, et

d     est un nombre compris entre 0 et 2, à condition que le catalyseur contienne comme Y au moins les éléments V et Nb.